(19) Europäisches Patentamt
European Patent Office
Office européen des brevets

(11) Veröffentlichungsnummer: **0 144 969**
**A2**

# (12) EUROPÄISCHE PATENTANMELDUNG

(21) Anmeldenummer: 84114773.9

(22) Anmeldetag: 05.12.84

(51) Int. Cl.⁴: **C 07 C 147/10**

(30) Priorität: 10.12.83 DE 3344676

(43) Veröffentlichungstag der Anmeldung: 19.06.85 Patentblatt 85/25

(84) Benannte Vertragsstaaten: DE FR GB IT

(71) Anmelder: **HOECHST AKTIENGESELLSCHAFT**
Postfach 80 03 20
D-6230 Frankfurt am Main 80(DE)

(72) Erfinder: **Lerch, Ulrich, Dr.**
Schwalbenweg 19
D-6238 Hofheim am Taunus(DE)

(72) Erfinder: **Englert, Heinrich Christian, Dr.**
Stormstrasse 13
D-6238 Hofheim am Taunus(DE)

(72) Erfinder: **Napierski, Bernd**
Flörsheimer Strasse 5
D-6234 Hattersheim am Main(DE)

## (54) Verfahren zur Herstellung von 2-Alkylsulfonyl-4-alkylphenolen.

(57) Verbindung der Formel I

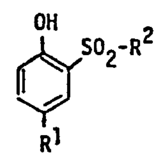

I

mit $R^1$ und $R^2$ gleich Alkyl werden erhalten, wenn man 4-Alkylanisole der Formel II

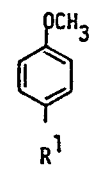

II

bei Temperaturen unterhalb von 0°C sulfochloriert und die resultierenden Verbindungen in einem Eintopfverfahren zu Verbindungen IV

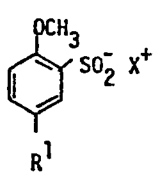

IV

reduziert, welche alkyliert werden. Etherspaltung mit HBr führt dann zu I.

EP 0 144 969 A2

Croydon Printing Company Ltd.

Verfahren zur Herstellung von 2-Alkylsulfonyl-4-alkylphenolen

2-Alkylsulfonyl-4-alkylphenole der Formel I

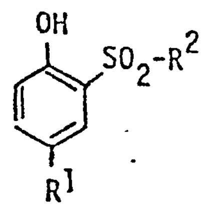 I

sind wertvolle Zwischenprodukte zur Herstellung von pharmazeutisch wirksamen Präparaten, insbesondere von Verbindungen mit salidiuretischen, blutdrucksenkenden und cytoprotektiven Eigenschaften, wie sie z.T. in der europäischen Patentanmeldung der Veröffentlichungsnummer 88 346 beschrieben sind.

Die vorliegende Anmeldung beschreibt ein neues Verfahren für die Herstellung von Verbindungen der Formel I

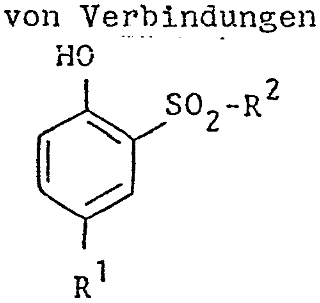 I,

worin bedeuten:

$R^1$ einen geradkettigen oder verzweigten Alkylrest mit 1 - 8 Kohlenstoffatomen oder Cycloalkyl mit 3 - 7 Kohlenstoffatomen,

$R^2$ einen geradkettigen oder verzweigten Alkylrest mit 1 - 5 Kohlenstoffatomen.

Wie in der europäischen Patentanmeldung der Veröffentlichungsnummer 88 346 beschrieben, werden Anisole der Formel II

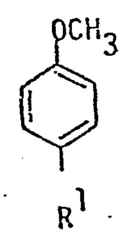 II

mit $R^1$ wie oben angegeben bei Temperaturen von 0 - 20°C sulfochloriert zu Verbindungen der Formel III

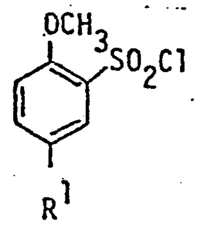

III

Unter den beschriebenen Reaktionsbedingungen werden jedoch Produkte von verhältnismäßig geringer Reinheit in schlechten Ausbeuten erhalten. Beispielsweise liefert die Sulfochlorierung von 4-tert. Butylanisol (II, R = t - Butyl) bei Temperaturen zwischen 0 und 10°C im 20 - 30 Mol-Maßstab nur Ausbeuten zwischen 25 und 38 %. Das erhaltene Produkt ist gefärbt und erfordert zusätzliche Reinigungsoperationen bzw. beeinträchtigt die Qualität der Folgeprodukte.

Überraschenderweise wurde gefunden, daß Sulfochloride der Formel III in guten Ausbeuten und sehr guter Qualität reproduzierbar erhalten werden, wenn die Sulfochlorierung zwischen -15 und -3°, vorteilhafterweise zwischen -10 und -4° durchgeführt wird und Nachrührzeiten von 1 - 7 Stunden, vorteilhafterweise zwischen 2 und 5 Stunden, eingehalten werden. Dabei ist es durchaus möglich, die Chlorsulfonsäure (bzw. die Chlorsulfonsäure in einem inerten Lösungsmittel) bei Temperaturen unterhalb von -15° zu der Lösung des Anisols II zulaufen zu lassen, oder umgekehrt das Anisol II zu der auf Temperaturen unterhalb von -15°C gekühlten Lösung der Chlorsulfonsäure zu dosieren, ohne die Gefahr einer plötzlich einsetzenden exothermen Reaktion beim Erwärmen der Mischung einzugehen, und erst danach auf die optimale Reaktionstemperatur von -10 bis -4° zu erwärmen.

Eine bevorzugte Ausführungsform des erfindungsgemäßen Verfahrens besteht darin, daß man zu einer Lösung des 4-Alkyl-anisols II in Methylenchlorid unterhalb von -5°C

Innentemperatur eine Lösung der 3- 4-fachen molaren Menge Chlorsulfonsäure in Methylenchlorid gibt. Nach Zugabe der molaren Menge Chlorsulfonsäure ist die Wärmetönung der Reaktion weitgehend abgeklungen, so daß die restliche Menge an Chlorsulfonsäure zügig zudosiert werden kann. Man rührt 3 - 5 Stunden bei -10 bis -4°C nach und gibt dann auf Eis oder Eiswasser. Die trübe Methylenchloridphase, die noch größere Mengen Wasser und Schwefelsäure enthält, wird abgetrennt und ggf. getrocknet. In einer bevorzugten Ausführungsform des Verfahrens wird jedoch die feuchte Methylenchloridphase ohne Trocknung, ggf. nach Zugabe von Wasser und zweckmäßigerweise in Gegenwart eines Entschäumers, wie z. B. Triisobutylphosphat (Etigal), i. Vak. vom organischen Lösungsmittel befreit. Man erhält dabei ein Gemisch des kristallinen Sulfochlorids III mit der Wasserphase. Das Produkt wird durch Filtration abgetrennt, sorgfältig mit Wasser gewaschen und ggf. bei Raumtemperatur getrocknet. Es kann aber auch im feuchten Zustand direkt für die folgende Reaktion eingesetzt werden.

Bei dem beschriebenen Vorgehen erhält man reproduzierbare Ausbeuten von 70 - 80 % an Sulfochlorid III.

Die Reduktion der Sulfochloride III zu den Sulfinaten IV

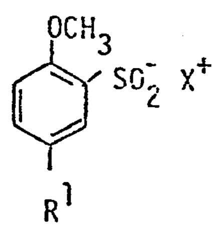 IV

(X= Alkalimetall) und deren Alkylierung zu den Sulfonen V

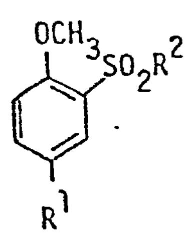 V

wird nach dem erfindungsgemäßen Verfahren im Sinn einer Eintopfreaktion durchgeführt. Überraschenderweise hat sich dabei gezeigt, daß die Ausbeuten gesteigert werden können, wenn man als Base bei dem Reaktions- und Alkylierungsschritt Kaliumhydroxyd und insbesondere Lithiumhydroxyd verwendet. Gegenüber dem bekannten Verfahren, bei dem Natriumhydroxyd eingesetzt wurde und die Sulfinsäuren IV (X = H) isoliert werden mußten, konnten so neben der Reduzierung des Aufwands an Arbeit, Zeit und Chemikalien die Ausbeute für die Stufenfolge III → V von 47 % auf 75 - 80 % gesteigert werden.

Sulfinatanionen IV sind ambidente Spezies, die von einem Alkylierungsmittel sowohl am Schwefelatom (Produkt Sulfon V)

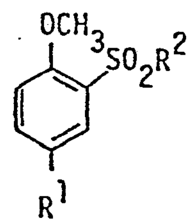

V

als auch an einem Sauerstoffatom (Produkt Sulfinsäureester VI)

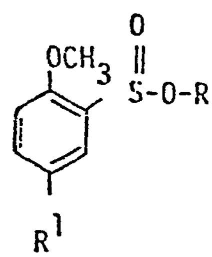

VI

angegriffen werden können. Nach der Theorie der harten und weichen Säuren und Basen (Chem. Rev. 75, 1 - 20, 1975) sollten weiche Reagentien wie Alkyljodide für die Alkylierung des weichen Schwefelatoms besonders günstig sein. Daher wurden zur Alkylierung der Sulfinsäuren nach dem in der europäischen Patentanmeldung der Veröffentlichungsnummer 88 346 beschriebenen Verfahren stehts Alkyljodide verwendet.

Es war deshalb sehr überraschend, als sich herausstellte, daß die härteren, also theoretisch ungünstigeren Alkylbromide bei der Alkylierung der Sulfinate IV vergleichbar hohe Ausbeuten an Sulfonen V ergeben wie die entsprechenden Alkyljodide. Zu den wirtschaftlichen Vorteilen und der verbesserten Arbeitssicherheit bei Verwendung der preiswerten und weniger toxischen Alkylbromide kommt hinzu, daß die Produkte V farblos und in großer Reinheit anfallen, während nach dem Alkyljodidverfahren verfärbte Sulfone V von geringerem Reinheitsgrad isoliert werden.

Nach dem erfindungsgemäßen Eintopfverfahren wird zur Herstellung der Alkylsulfonylverbindungen der Formel V das rohe Sulfochlorid III in eine Mischung, bestehend aus 1 - 2 Mol Natriumsulfit, 1,5 - 3 Mol einer Base, wie z. B. einem Alkalihydroxyd wie Kaliumhydroxyd oder vorzugsweise Lithiumhydroxyd und einem geeigneten Lösungsmittel, eingetragen.

Als Lösungsmittel kommen infrage Wasser oder eine Mischung von Wasser mit Aceton, 2-Butanon, Tetrahydrofuran, Dimethoxyethan, Acetonitril, Dimethylformamid, Dimethylsulfoxid oder einem niedermolekularen Alkohol wie Methanol oder Isopropanol. Man läßt 15 Minuten bis 3 Std. bei 0 - 50° reagieren. Wenn das Sulfochlorid vollständig verbraucht wird, gibt man 1 - 3 Mol (bezogen auf das eingesetzte Sulfochlorid) Alkylbromid zur Reaktionsmischung und läßt 3 - 15 Stunden bei 0 - 50° einwirken.

In einer besonders bevorzugten Ausführungsform des Verfahrens gibt man das Sulfochlorid zu einer Mischung von 2 - 3 Mol Lithiumhydroxyd und 1,1 bis 1,3 Mol Natriumsulfit in Wasser/Aceton als Lösungsmittel. Unter leichter Kühlung läßt man bei etwa 20 bis 35° reagieren, bis das Sulfochlorid vollständig reduziert ist, wofür gewöhnlich

15 - 60 Minuten benötigt werden. Danach dosiert man 1,5 - 2,5 Mol des entsprechenden Alkylbromids zu und läßt im Fall von $R^2 = CH_3$ bei Zimmertemperatur reagieren. Bei höheren Alkylbromiden muß die Reaktionsmischung ggf. erwärmt werden. Anschließend wird das Reaktionsgemisch auf Wasser gegeben, wobei das Sulfon V auskristallisiert. Man kann auch durch Verdampfen des Acetons aus der Reaktionsmischung das Produkt ausfällen und durch Filtration isolieren. Es ist für die weiteren Reaktionsschritte genügend rein und kann ohne Trocknung direkt für die folgende Etherspaltung verwendet werden.

Verbindungen der Formel I, bei denen $R^1$ einen tert. Butylrest bedeutet, sind zur Herstellung von pharmazeutisch wirksamen Präparaten von besonderem Interesse. Es ist aber bekannt, daß tert. Alkylgruppen von Lewis-Säuren unter verschärften Reaktionsbedingungen von benzolaromatischen Verbindungen abgespalten werden und schwer zu reinigende Substanzgemische entstehen können. Tatsächlich ergeben Versuche von Etherspaltungen an Verbindungen der Formel V mit $R^1$= tert. Butyl mit Reagentien wie $AlCl_3$ oder Jodwasserstoffsäure unbrauchbare Substanzgemische. Daher war es sehr überraschend, daß auch Ether der Formel V, worin $R^1$ eine tert. Alkylgruppe bedeutet, mit HBr in guter Ausbeute zu Phenolen der Formel I gespalten werden können, obwohl hierfür ein mehrstündiges Kochen in wäßriger Bromwasserstoffsäure oder in einem Bromwasserstoff/Wasser/Eisessig-Gemisch erforderlich ist. Gegenüber der in der europäischen Patentanmeldung der Veröffentlichungsnummer 88 346 beschriebenen Methode der Etherspaltung mit Pyridiniumhydrochlorid, die bei Temperaturen zwischen 190 und 220° durchgeführt wird, hat das erfindungsgemäße Verfahren mit HBr den Vorteil, daß es technisch viel einfacher handhabbar ist, ein Produkt höherer Reinheit liefert und die Ausbeuten mit 75 - 85 % wesentlich höher liegen als bei dem Pyridiniumhydrochlorid-Verfahren (40 - 50 %).

Das beim zweiten in der europäischen Patentanmeldung der Veröffentlichungsnummer 88 346 beschriebenen Verfahren zur Etherspaltung der Anisolderivate V verwendete Bortribromid ist toxisch, sehr feuchtigkeitsempfindlich und daher technisch schlechter handhabbar als Bromwasserstoffsäure. Angesichts der hohen Marktpreise für Bortribromid bringt das HBr-Verfahren auch sehr deutliche wirtschaftliche Vorteile.

Im einzelnen führt man die Etherspaltung nach dem erfindungsgemäßen Verfahren durch, indem man die Anisolderivate der Formel V mit konzentrierter wäßriger Bromwasserstoffsäure, vorteilhafterweise in Gegenwart von Eisessig oder einer Lösung von Bromwasserstoffgas in Eisessig, unter Rückfluß erhitzt. Wenn die Reaktion weitgehend beendet ist, was nach etwa 5 - 12 Stunden der Fall ist, gibt man das Reaktionsgemisch auf eine Mischung von Wasser oder Eis und einem wenig polaren, mit Wasser nicht mischbaren organischen Lösungsmittel, wie z. B. Toluol, Diisopropylether, tert. Butylmethylether, Ethylacetat oder Butylacetat. Die organische Phase wird anschließend mit einem wäßrigen Alkalihydroxyd extrahiert, vorteilhafterweise mit Kaliumhydroxyd, das in Wasser leichter lösliche Phenolate ergibt. Aus der extrahierten organischen Phase kann man geringe Mengen an nicht gespaltenem Anisolderivat V wiedergewinnen, während man durch Ansäuern der wäßrigen, alkalischen Extrakte die erwünschten Verbindungen I erhält. Das Ansäuern erfolgt mit beliebigen organischen oder anorganischen Säuren, vorteilhafterweise jedoch mit Salzsäure oder Schwefelsäure, wobei die Verbindungen der Formel I auskristallisieren. Nach der Filtration, Waschen mit Wasser und Trocknen erhält man die Phenole I in einem hohen Reinheitsgrad und einer Ausbeute von 80 - 90 %, bezogen auf die Stufe der Etherspaltung.

Beispiel 1 (Zwischenstufe)

5-t-Butyl-2-methoxy benzolsulfonsäurechlorid
(III, $R^1 = t - C_4H_9$)

Unter einer Stickstoffatmosphäre läßt man zu einer Lösung von 5,25 kg 4-Butylanisol in 12,7 l Methylenchlorid eine Lösung von 6,7 l Chlorsulfonsäure in 8 l Methylenchlorid zulaufen, wobei die Innentemperatur unterhalb von -5°C gehalten wird. Nach Zugabe des ersten Drittels der Chlorsulfonsäurelösung ist die stark exotherme Reaktion abgeklungen, so daß die Zugabe der restlichen Chlorsulfonsäure ziemlich rasch erfolgen kann. Die Reaktionsmischung wird danach 4,5 Stunden bei -5 bis -10°C nachgerührt und auf 80 l Eiswasser im Scheidetrichter gegeben. Die Mischung wird gut verrührt und die milchige organische Phase i. Vak. nach Zugabe von 5 g Tributylphosphat (Entschäumer) von Methylenchlorid befreit. Das Produkt kristallisiert aus den Wasserresten aus und wird abgesaugt oder geschleudert und sorgfältig mit Wasser gewaschen. Es kann in feuchtem Zustand direkt für die nächste Umsetzung verwendet oder i. Vak. bei Zimmertemperatur getrocknet werden.

Ausbeute: 6,18 kg (73,6 % d. Th.)

Beispiel 2 (Zwischenstufe)

4-t-butyl-2-methylsulfonylanisol
(V, $R^1 = t-C_4H_9$, $R^2 = CH_3$)

Zu einer Lösung von 2,25 kg $LiOH \cdot H_2O$ und 2,88 kg Natriumsulfit in 24 l Wasser gibt man 24 l Aceton und trägt innerhalb von 30 Minuten unter Stickstoff 5 kg des rohen 5-t-Butyl-2-methoxybenzolsulfonsäurechlorids ein, wobei die Innentemperatur 30° betragen soll. Man läßt 15 Minuten nachrühren und kontrolliert im Dünnschichtchromatogramm auf vollständige Reduktion.

Wenn kein Sulfochlorid mehr vorhanden ist, werden bei einer Innentemperatur von 20 - 30° 3,6 kg Methylbromid-Gas eingeleitet. Anschließend läßt man über Nacht (etwa 17 Stunden) bei Zimmertemperatur rühren. Das Reaktionsgemisch wird anschließend in 100 l Eiswasser eingetragen, wobei das farblose Produkt auskristallisiert. Es wird abgesaugt, mit Wasser gewaschen und kann in feuchtem Zustand für die nächste Stufe eingesetzt werden. Nach Trocknen i. Vak. erhält man 3,55 kg farblose Kristalle (77 % d. Th.).

Beispiel 3

4-t-Butyl-2-methylsulfonylphenol
(I, $R^1$= t-$C_4H_9$, $R^2$= $CH_3$)

4 kg 4-t-Butyl-2-methylsulfonylanisol in 3 l HBr/Eisessig (33 %ig) und 7,5 l 48 %iger wäßriger HBr werden etwa 10 Stunden zum Sieden erhitzt, bis das Ausgangsmaterial fast vollständig verbraucht ist. Danach wird das Reaktionsgemisch unter kräftigem Rühren auf eine Mischung von etwa 20 l Eiswasser und 15 l Toluol gegeben. Die Toluolphase wird abgetrennt und mit einer Lösung von 1,5 kg Kaliumhydroxyd in 14 l Wasser ausgerührt. Die wäßrige Phase wird mit der doppelten Menge Eis versetzt und danach unter Rühren mit konz. Salzsäure angesäuert (pH 1 - 2). Das auskristallisierte Produkt wird abgesaugt oder geschleudert und mit Wasser gewaschen.

Ausbeute 3,0 - 3,4 kg (80 - 90 % d. Th.)

<u>Patentansprüche:</u>

HOE 83/F 262

1. Verfahren zur Herstellung von 4-Alkyl-2-alkylsulfonylphenolen der Formel I

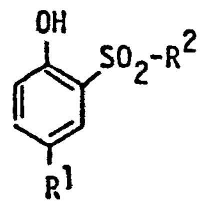

I

in der $R^1$ einen geradkettigen oder verzweigten Alkylrest mit 1 - 8 Kohlenstoffatomen oder Cycloalkyl mit 3 - 7 Kohlenstoffatomen, $R^2$ einen geradkettigen oder verzweigten Alkylrest mit 1 - 5 Kohlenstoffatomen bedeuten, dadurch gekennzeichnet, daß man 4-Alkylanisole der Formel II

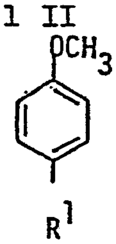

II

bei Temperaturen unterhalb von 0° sulfochloriert zu Verbindungen der Formel III

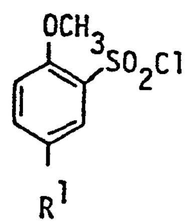

III

diese in einem Eintopfverfahren zu Verbindungen der Formel IV reduziert

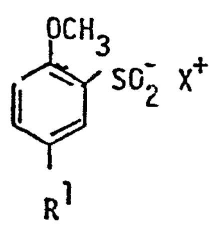

IV

und weiter zu Verbindungen V alkyliert

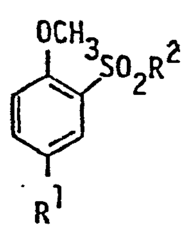

V

und schließlich mit Bromwasserstoffsäure die Etherspaltungen durchführt, wobei Verbindungen der Formel I entstehen.

2. Verfahren gemäß Anspruch 1, dadurch gekennzeichnet, daß die Sulfochlorierung von Verbindungen der Formel II zu Verbindungen der Formel III bei Temperaturen von -15 bis -3°C, vorzugsweise bei -10° bis -4°C durchgeführt wird.

3. Verfahren gemäß Anspruch 1, dadurch gekennzeichnet, daß man nach Durchführung der Sulfochlorierung in Methylenchlorid und Aufarbeitung der Reaktionslösung mit Eis oder Wasser die feuchte emulsionartige organische Phase ohne Trocknung direkt eindampft, vorzugsweise in Gegenwart eines Entschäumers.

4. Verfahren gemäß Anspruch 1, dadurch gekennzeichnet, daß die Sulfochloride III im Sinne einer Eintopfreaktion in die Sulfone der Formel V umgewandelt werden, ohne daß die intermediär auftretenden Sulfinsäurederivate IV isoliert werden.

5. Verfahren gemäß Anspruch 1, dadurch gekennzeichnet, daß bei der Überführung von Verbindungen der Formel III in die Verbindungen IV und V, Kaliumhydroxyd oder insbesondere Lithiumhydroxyd als Base verwendet werden.

6. Verfahren gemäß Anspruch 1, dadurch gekennzeichnet, daß die Sulfinsäuren IV mit Alkylbromiden zu den Sulfonen der Formel V alkyliert werden.

7. Verfahren gemäß Anspruch 1, dadurch gekennzeichnet, daß man die Anisolderivate der Formel V durch Erhitzen mit konzentrierter wäßriger Bromwasserstoffsäure, ggf. in Gegenwart von Eisessig oder Bromwasserstoff/Eisessig, zu Phenolen der Formel I spaltet.

8. Verfahren gemäß Anspruch 1, dadurch gekennzeichnet, daß nach der Etherspaltung mit HBr die Verbindungen der Formel I dadurch isoliert und gereinigt wird, daß man die Reaktionslösung zwischen Wasser und einem mit Wasser nicht mischbaren, wenig polaren organischen Lösungsmittel verteilt, die organische Phase mit einem Alkalihydroxyd, vorteilhafterweise verdünnter Kalilauge, extrahiert und durch Ansäuern der wäßrigen Phase das Phenol der Formel I erhält.